# EUROPEAN PATENT SPECIFICATION

(11) **EP 2 690 445 B1**
(45) Date of publication and mention of the grant of the patent: **03.05.2023**
(21) Application number: 12764053.0
(22) Date of filing: 23.03.2012
(51) Int. Cl.: G01N 35/02, G01N 35/10, G01N 35/04, G01N 35/00, G01N 33/53

(54) **APPARATUS FOR AUTOMATIC ANALYSIS AND SAMPLE ANALYSIS METHOD THEREOF**
VORRICHTUNG ZUR AUTOMATISCHEN ANALYSE UND PROBENANALYSEVERFAHREN DAFÜR
APPAREIL POUR UNE ANALYSE AUTOMATIQUE ET PROCÉDÉ D'ANALYSE D'ÉCHANTILLONS À L'AIDE DE CELUI-CI

(30) Priority: 25.03.2011 CN 201110074104
(43) Date of publication of application: 29.01.2014
(73) Proprietor: Shenzhen Mindray Bio-Medical Electronics Co., Ltd., Guangdong 518057 (CN)
(72) Inventor: ZHANG, Zhen, Guangdong 518057 (CN); ZHOU, Peng, Guangdong 518057 (CN); WANG, Jun, Guangdong 518057 (CN); XIE, Chuanfen, Guangdong 518057 (CN); WANG, Hai, Guangdong 518057 (CN)
(74) Representative: KIPA AB
(86) International application number: PCT/CN2012/072950
(87) International publication number: WO 2012/130107

(56) References cited:
- EP-A2- 0 833 278
- WO-A2-2010/059569
- CN-A- 1 053 682
- CN-A- 101 393 225
- CN-A- 101 566 624
- CN-A- 101 726 616
- CN-A- 101 965 518
- US-A- 4 857 471
- US-A- 5 212 094
- US-A- 5 380 487
- US-A- 5 985 672
- US-A- 5 985 672
- US-A1- 2007 189 925
- US-A1- 2009 081 794
- US-A1- 2009 150 081

## Description

### TECHNICAL FIELD

This disclosure relates to an automatic analyzer and sample analysis method. Specifically, this disclosure relates to a system structure and sample analysis method of an automatic analyzer.

### BACKGROUND

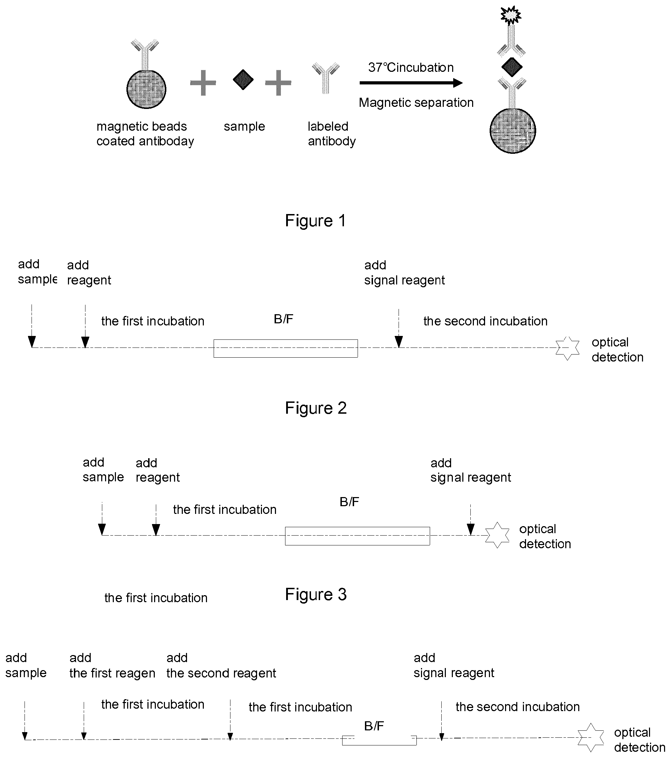

An immunoassay analyzer is a highly sensitive and highly specific kind of analyzer. In the clinical experiments, it is usually used to test or analysis targets such as blood, urine, or other body liquid. Conventional immunoassay analyzers use many different implementation principals, for example, chemiluminescence, electrochemical luminescence, etc. As shown in figure 1 , for non homogeneous chemiluminescence the primary working principal is as follows: if detecting object substance in the sample, a magnetic bead reagent is formed by covering the magnetic bead with an antibody/antigen, a labeling reagent is formed by marking the antibody with a given marker (there are usually multiple components of reagents for analyzing a certain kind of analysis items, for example: magnetic bead reagent components, mark reagent components, etc; different kinds of components of the reagent can be put separately in different reagent vessels or in different cavities of the same reagent vessel). When testing, firstly the sample containing the analyte is mixed together with a magnet bead reagent, a labeling reagent and other reagents, in order to form a sample and reagent reaction solution (called a reaction solution for short) and under certain conditions, reaction complexes are formed by an incubation reaction. Then using bound -free technology (referred to as B/F for short) unbound markers and other reagents, sample of reaction system are removed. A signal reagent then is added and, then a marker of reaction complexes is reacted with the signal reagent (or catalytic signal reagent), and generate light. The signal reagent can be of different types such as, for example, a luminescent substrate solution, a pre-excitation liquid, an excitation liquid and a luminescence enhancement solution, etc. There are many kinds of methods for specific coating and B/F. In addition to the magnetic bead method, methods also including coating an antibody on the wall of reaction vessel, plastic beads, etc.

For different characteristics of test items, a common immunoassay analyzer needs to support several different kinds of test processes.

### (1) One step protocol

This is illustrated in 2. The one step protocol means a reagent is added once when testing; it is the most simple test method. A reagent and a sample are added to a reaction vessel, and mixed together so as to form a reaction solution. Thereafter the mixed reaction solution is incubated under thermostatic conditions B/F is then performed and signal reagent is added to the reaction vessel when B/F is finished. Optical detection of the reaction vessel is performed after the signal reagent has been added to the reaction vessel and incubated for a certain time. Some kinds of tests may permit optical detection immediately and do not need incubation after adding the signal reagent, for example, chemiluminescence test which is based on electrochemical luminescence or flash system. Please refer to figure 3.

### (2) Two steps one B/F protocol

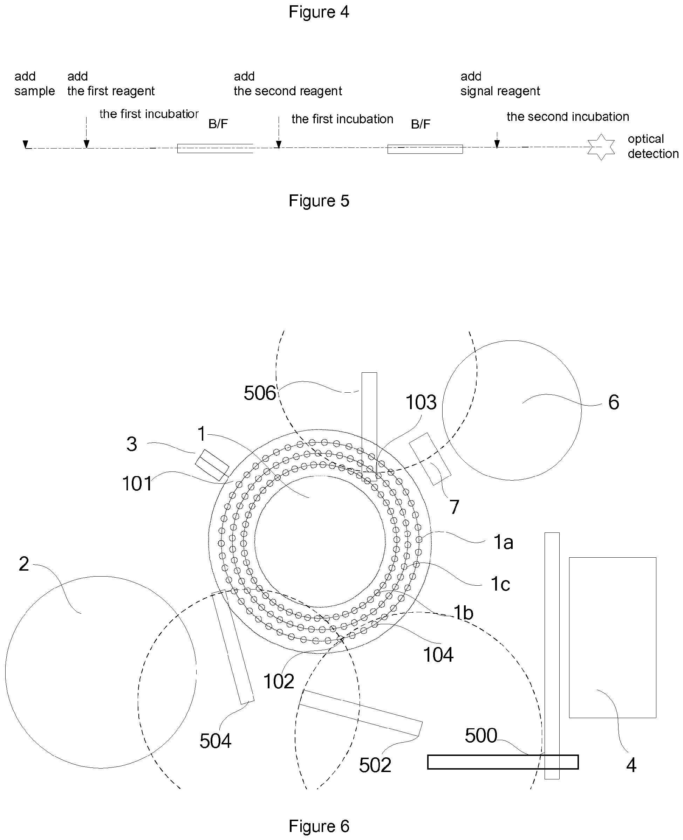

As shown in figure 4, a reagent (called a first reagent but may include several kinds of components) and a sample are added to the reaction vessel and mixed to form reaction solution. The mixture in the reaction vessel is incubated under thermostatic conditions for a certain time. Then a reagent (called a second reagent but can include several kinds of components) is added to the reaction vessel and mixed with the reaction solution which is a mixture of the first reagent and the sample, then the mixture in the reaction vessel is incubated under thermostatic conditions for a certain time. The B/F is executed after incubation, then the signal reagent is added to the reaction vessel when B/F is finished. The reaction vessel with the added signal reagent is then incubated under thermostatic conditions for a certain time. Optical detection is then performed. As mentioned above, some kinds of test may perform optical detection directly and do not need incubation after adding the signal reagent.

### (3) Two step two B/F protocol

Please refer to figure 5. Some reagent (called a first reagent but may include several kinds of components) and a sample are added to a reaction vessel and mixed to form a reaction solution. B/F is executed after the mixed reaction solution has been incubated under thermostatic conditions. A reagent (called the second reagent but may include several kinds of compositions) is then added to the reaction vessel and mixed with the reaction solution which is a mixture of the first reagent and the sample. The mixture in the reaction vessel is then incubated under thermostatic conditions for a certain time. B/F is executed after incubation, then a signal reagent is added to the reaction vessel after B/F is finished. After the signal reagent has been added to the reaction vessel the vessel is incubated under thermostatic conditions for a certain time. Optical detection is then performed. As mentioned above, some tests may permit optical detection to be performed directly and do not require incubation after adding the signal reagent.

In the different testing processes described above, the incubation time is different according to the assay. So as to obtain accurate test results and support more test items, an immunoassay analyzer need to support a very flexible test method.

In conventional immunoassay analyzers, incubation, detection and B/F functions are set in the same disk, but there are some defects for this design. (1) Flexible rotation is restricted because of B/F, so the incubation time is fixed and difficult to adapt to reaction characteristics of different test items. (2) Because incubation, detection and B/F are in the same disk, the disk is too big, and the structure of the disk is complex, so the manufacturing process is difficult. (3) In order to support two step one B/F protocol, two B/F equipments are needed, and then the cost is high. (4) Incubation requires needs the control of a stable disk temperature, but the B/F operation will cause the temperature of the disk to fluctuate, which results in unstable test results. In another conventional immunoassay analyzer, the incubation of the reaction solution, B/F, the incubation after adding signal reagent, and the optical detection functions are done separately in different structural units; however, there are also some defects for this design. (1) The cost is high, the structure is complex, and the size of the whole analyzer is large. (2) Because the incubation of the reaction solution, the incubation after adding signal reagent and the optical detection function are done separately in different structural units, the energy efficiency is low. Also it requires coordination between the different units, so the control is complex, the test steps are many, and the system structure is complex.

US 2007/0189925 discloses a system and method for the simultaneous detection of multiple analytes in a sample. The detection system includes a housing that holds a reagent carousel rotatably coupled thereto. Further included in the housing is an incubator carousel rotatably coupled thereto. US 5,380,487 discloses an analyzer which permits clinical analysis of samples for multiple analytes with a variety of assay protocols in a multiple chronology sequence while operating on a predetermined fizzed length cycle method of timing control. In this analyzer, assay resources (e.g. an incubator belt, a wash station, and a signal detection apparatus) are assigned fixed operating sequences which begin and end within a time cycle of fixed duration. WO 2010/059569 discloses an analyzer for analyzing at least one or more samples for a desired trait. Analyzer generally includes a housing, an incubation assembly located at least partially within the housing, a carousel assembly located at least partially within the incubation assembly and movable relative to the incubation assembly, and a probe assembly. EP 0 833 278 discloses a data carrier for use with a diagnostic instrument. US 5,985,672 discloses a pre-processor for use in performing heterogeneous immunoassays on samples for analytes in the sample employing concentrically positioned incubating and processing carousels. A single transfer station permits reaction vessels containing sample and reagents to be moved between the carousels. US 2009/081794 discloses a sample analyzer which enables to confirm an analysis remaining time for each sample and a total analysis remaining time for all samples set in the analyzer. The sample analyzer comprises primary and secondary reaction members. During a sample analysis process, the cuvette containing the sample and reagents was conveyed from the primary reaction member to the secondary reaction member, then conveyed from the secondary reaction member to the detector module. In particular, it discloses an automatic analyzer, including: a reaction disk for holding a reaction vessel and carrying the reaction vessel to a predetermined operation position, the operation position including a dispensing operation position and a detection operation position; a detection unit; a B/F unit, for removing unbound components of reaction system; a dispensing unit, for dispensing a reagent and/or a sample to the reaction vessel; a reaction vessel supply unit, for holding reaction vessels; a conveying unit, for conveying the reaction vessel between the reaction disk, the B/F unit and the reaction vessel supply unit, wherein the conveying unit includes a first convey unit and a second convey unit, the first conveying unit for conveying the reaction vessel between the reaction disk and the B/F unit, the second conveying unit for conveying the reaction vessel between the reaction disk and the reaction vessel supply unit; wherein the reaction disk and the B/F unit are independent structures, and the B/F unit is disposed outside and separated from the reaction disk with sufficient spacing to avoid cross-influence between the B/F unit and the reaction disk, and the reaction disk is provided with a first incubation position and a second incubation position which are distinct from each other, wherein the reaction disk is configured to incubate a solution with a reagent and a sample in the reaction vessel to the first incubation position to form a reaction solution, the first conveying unit is configured to convey the reaction solution in the reaction vessel by the first conveying unit to the B/F unit to form a cleaned reaction solution.

### DISCLOSURE OF THE INVENTION

In order to solve the problem of the prior art, the invention provides an automatic analyzer and sample analysis method, which can effectively prevent the incubation and B/F processes influencing each other, so permitting more flexible testing and a simpler structure.

According to one aspect of the present invention, the invention provides an automatic analyzer as claimed in claim 1.

According to another aspect of the present invention, the invention provide a sample analysis method as claimed in claim 8.

In accordance with the present invention the reaction unit which performs the incubation function and the B/F unit which has a cleaning and separation function are arranged separately, so the incubation of the reaction vessel and the B/F are finished in different units, thus preventing the incubation and B/F functions from influencing each other. At the same time, the automatic analyzer tests are more flexible, and as the first incubation, the detection and the second incubation are all finished in the reaction unit, the system structure will be simple, and the operating efficiency will be improved.

### BRIEF DESCRIPTION OF THE DRAWINGS

Figure 1 illustrates principle drawing of immunoassay analyzer;
Figure 2 illustrates one step protocol;
Figure 3 illustrates another one step protocol;
Figure 4 illustrates two steps one B/F protocol;
Figure 5 illustrates two steps two B/F protocol;
Figure 6 illustrates a schematic diagram of the first embodiment of the automatic analyzer for the present invention;
Figure 7 illustrates flow chart of one step protocol;
Figure 8 illustrates flow chart of two steps one B/F protocol;
Figure 9 illustrates flow chart of two steps two B/F protocol;
Figure 10 illustrates schematic diagram of the first embodiment of the present reaction disk;
Figure 11 illustrates schematic diagram of the second embodiment of the present reaction disk;
Figure 12 illustrates schematic diagram of the third embodiment of the present reaction disk;
Figure 13 illustrates schematic diagram of the forth embodiment of the present reaction disk;
Figure 14 illustrates schematic diagram of the fifth embodiment of the present reaction disk;
Figure 15 illustrates schematic diagram of the sixth embodiment of the present reaction disk;
Figure 16 illustrates schematic diagram of another embodiment of the present reaction unit;
Figure 17 illustrates schematic diagram of the second embodiment of the automatic analyzer not being part of the invention;
Figure 18 illustrates schematic diagram of the third embodiment of the present automatic analyzer;
Figure 19 illustrates schematic diagram of the forth embodiment of the automatic analyzer not being part of the invention;
Figure 20 illustrates schematic diagram of the fifth embodiment of the automatic analyzer not being part of the invention;
Figure 21 illustrates schematic diagram of the sixth embodiment of the automatic analyzer not being part of the invention.

### DETAILED DESCRIPTION

A detailed description of systems and methods consistent with embodiments of the present disclosure is provided below.

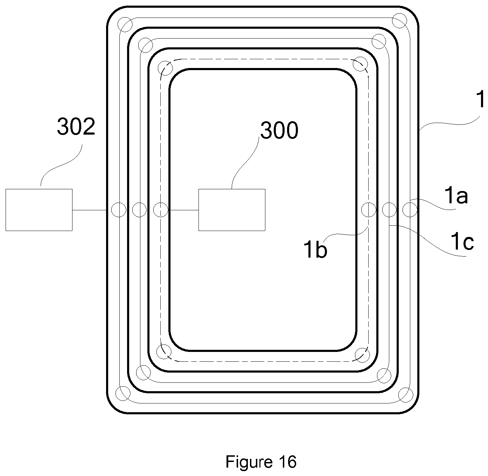

The present automatic analyzer includes: a reaction unit for holding a reaction vessel and carrying the reaction vessel to a predetermined operating position, the operating position including a detection operating position; a detection unit for detecting a substance in the reaction vessel of the reaction unit in the detection operating position; a B/F unit for removing unbound components of the reaction system; a dispensing unit for dispensing a reagent and/or a sample to the reaction vessel; wherein the reaction unit has an incubation position for incubating a solution in the reaction vessel. The reaction unit may be circular, as show in figure 6, figure 10-15, it may also be rectangular, as show in figure 16 . In a preferred form the reaction unit is a reaction disk, so, in order to describe simply, it is described by a circular reaction disk.

Because the technology of the antibody coated to solid phase is different, the general aim of B/F is to remove other unnecessary compositions of the reaction system such that the reaction reactant remains. If the technology of magnetic bead coating is adopted, use of a magnetic field is required for gathering the magnetic beads in the reaction solution on the wall of the reaction vessel, after which other residue is removed. If reaction vessel surface coated antibody technology is adopted, the reaction vessel can be cleaned directly.

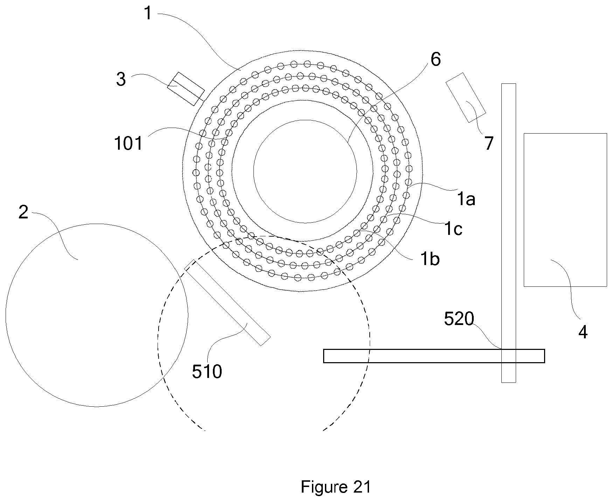

To finish incubation, the automatic analyzer usually controls the incubation temperature with a temperature control apparatus, however, the B/F unit may influence the temperature because of the B/F flow. The present reaction disk has an incubation function, but the B/F unit is disposed outside the reaction disk. By arranging the incubation unit and the B/F unit separately, the incubation temperature is more stable, and the influence of B/F on the incubation temperature control is avoided. Those skilled in the art will understand that, in examples not forming part of the present invention, the B/F unit may also be arranged at an inner portion of the reaction disk, please refer to figure 21. This is possible as long as the spacing of reaction disk is sufficient to avoid influence between the B/F and reaction disk incubation. Arrangement of the B/F unit and the reaction vessel can be designed according to needs; the reaction vessel is conveyed between the B/F unit and the reaction disk by a conveying unit. According to the invention the incubation of the reaction vessel and the B/F are performed separately in the different units, thereby preventing influence between the incubation and B/F, so allowing more flexibility in the analysis tests, which improves the efficiency of the analyzer.

Reference is made to figure 6, which is an embodiment for a one-step method testing process. The automatic analyzer includes a reaction disk 1, a reagent disk 2, a detection unit 3, a reaction vessel supply unit 4, a second conveying unit 500, a sample dispensing unit 502, a reagent dispensing unit 504, a first conveying unit 506, a B/F unit 6 and a mixing unit 7. The reaction disk is circular, including at least one ring, the reaction disk has distributed thereon a number of locations, these locations may be holes or slots, or the like, which are used for incubating and/or holding the reaction vessels, and conveying the reaction vessels to a predetermined operation position, which is convenient for other units to do corresponding operations. The operation position is a specific position which a reaction vessel on the reaction disk can be conveyed to, for example, a detection operation 101 position of the detection unit 3 for detecting an analyte in the reaction vessel, a dispensing operation position 102 of the dispensing unit for dispensing a sample and/or a reagent, a conveying operation position 103, 104, etc, of the conveying unit for conveying a reaction vessel. Those skilled in the art will understand that additional or fewer operation positions may be provided than those described above. In addition, the operation positions described above may overlap or include other operating positions, for example, operation positions 102 and 104 may be the same position, so that the dispensing operation position 102 may include a sample dispensing operation position and a reagent dispensing position, etc. The reagent disk 2 is used for carrying a reagent vessel, for example, a reagent bottle. Detecting unit 3 is used for detecting an analyte in the reaction vessel in the reaction disk. The detecting unit may be a photometer, which obtains an analyte concentration by detecting light intensity. The reaction vessel supply vessel 4 is used for holding reaction vessels, such as a cuvette. The conveying unit includes a first conveying unit 506 and a second conveying unit 500. The second conveying unit 500 is used for conveying a reaction vessel between reaction disk 1 and the reaction vessel supply unit 4. The dispensing unit includes sample dispensing unit 502 and reagent dispensing unit 504. The sample dispensing unit is a sample needle which is used for drawing and discharging a sample. Reagent dispensing unit is a reagent needle which is used for drawing and discharging a reagent. The trajectory of the first conveying unit intersects with the reaction disk 1, the B/F unit 6 and passes the mixing unit 7. The first conveying unit 506 is used for conveying the reaction vessel between the reaction disk 1 and B/F unit 6, and also be used for conveying the reaction vessel between the reaction disk 1 and mixing unit 7. The B/F unit 6 is used for removing the unbound components of reaction system. The B/F unit 6 is located outside the reaction disk 1, that is, the B/F unit and the reaction disk are arranged independently. The mixing unit 7 is used for mixing the reaction solution, however, some sample analysis methods do not need mixing.

The Reaction vessel supply unit 4 supplies the reaction vessel to the automatic analyzer. A new reaction vessel is conveyed to conveying operation position 104 by the second conveying unit 500.

The present reaction disk 1, mixing unit 7 and B/F unit 6 are located in the orbit of the first conveying unit 506. The reaction vessel may be conveyed between reaction disk 1 and mixing unit 7 easily, it also is conveyed between reaction disk 1 and B/F unit 6. Certainly, two conveying units may also be provided, one to convey the reaction vessel between the reaction disk 1 and B/F unit 6, and the other to convey the reaction vessel between the reaction disk 1 and mixing unit 7.

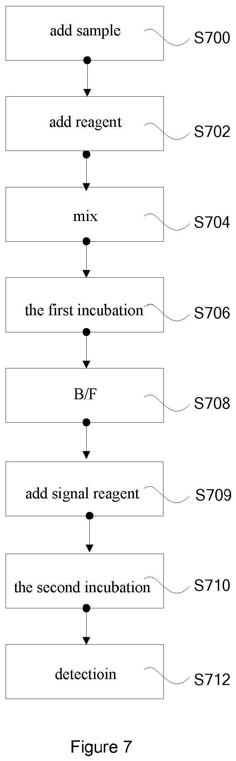

Reference will now be made to figures 6 and 7, which illustrates the whole analyzer work flow. The reaction disk 1 moves an empty location to operation position 104. Thereafter, the reaction vessel is conveyed to the reaction disk 1 by the second conveying unit 500 and, the reaction disk 1 then conveys reaction vessel to the dispensing operation position102. In step S700, the sample is added to the reaction vessel by the sample dispensing unit 502 in dispensing operation position102. In step S702, the reagent is added to the reaction vessel by the reagent dispensing unit 504 in dispensing operation position 102 and the reaction disk1 conveys the reaction vessel to conveying operation position 103. In step S704, the first conveying unit 506 conveys the reaction vessel, which contains the sample and the reagent, to mixing unit 7 for mixing. In step S706, the reaction vessel, in which mixing is finished , is conveyed to middle ring 1 c and/or inner ring 1 b of the reaction disk 1 for incubation. The first incubation position is defined as the position the reaction disk adopts for the first incubation. In step S708, after termination of the first incubation time, the reaction disk 1 conveys the reaction vessel back to the conveying operation position 103, then, the reaction vessel is conveyed to B/F (cleaning/separation) unit 6 by the first conveying unit 506. After several cycles, the reaction vessel is moved by B/F unit 6, and then the reaction vessel is cleaned and separated by the B/F unit 6. There are many kinds of B/F methods, which depend on the coating technology adopted. For example, in a magnetic B/F method, magnetic beads are coated with an antibody or antigen. The antibody or antigen can also be applied to the surface of the reaction vessel, or to another solid phase surface, which corresponds to different B/F methods. In step S709, a signal reagent is added to the reaction vessel. The signal reagent may be added to the reaction vessel in the B/F unit 6, which can also convey the reaction vessel to reaction disk 1 firstly, and then add the signal reagent to the reaction vessel, which can also convey the reaction vessel to certain position out of reaction disk 1 and B/F unit 6 firstly, then add the signal reagent to the reaction vessel, for ease of presentation, hereinafter just expressed adding the signal reagent to reaction vessel in B/F unit for example. There are also many different luminous methods. In the present embodiment, the method of marker catalyzing signal reagent luminescence is adopted. The method adopted may also be by the reaction of marker and signal reagent, or use the application of additional conditions, for example, illuminating by the function of additional electric field, magnetic field, optical excitation, etc, so as to detect the analyte concentration of the sample correspondingly. For chemiluminescence analyzers, the general operation flow includes dispensing reagent and sample, mixing the reaction solution, incubation, B/F (cleaning/separating), adding signal reagent and detecting. Some analysis methods do not require mixing. In step S710, after the reaction vessel with added signal reagent in the B/F unit 6 moves to the orbit of the first conveying unit 506 again, the reaction vessel is conveyed to the outer ring 1 a of the reaction disk 1 by the first conveying unit 506 for incubation. , This incubation after adding signal reagent is defined as the second incubation position. Alternatively, in other tests where incubation after the addition of a signal reagent is not necessary, the reaction vessel 1 is conveyed to operation 101 for detecting light, for example, chemiluminescence test which is based on electrochemiluminescence or flash system, etc. In Step 712, the reaction disk 1 conveys reaction vessel to detection operation position 101, the detection unit 3 detects an analyte in the reaction vessel, and the detecting signal may be an optical signal of the solution in the reaction vessel. After detection, the reaction vessel is conveyed to conveying operation position 104 by the reaction disk 1, then, the reaction vessel is abandoned by the second conveying unit 500, so the one step protocol is finished. Those skilled in the art will understand that in other examples, the sample may be added first, in order to facilitate the description, sample is added firstly for example to express at present.

Those skilled in the art will understand that, in figure 6, the dispensing unit includes sample dispensing unit 502 and reagent dispensing unit 504 and conveying unit includes the first conveying unit 506 and the second conveying unit 500. In the specific implementation of the process, when considering the essential factors of optimal combination of test efficiency, the whole size of the analyzer and the cost, etc, the number of dispensing units and conveying units may be increased or reduced, the number of dispensing unit and conveying unit may flexibly combined, for example, just one dispensing unit and just one conveying unit, or three dispensing units and two conveying units, etc.

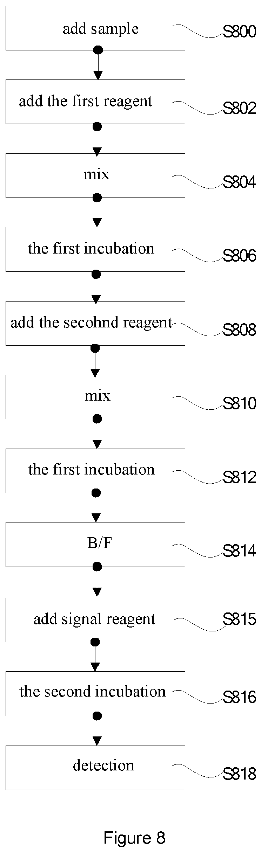

Reference is now made to figure 8, where, in another embodiment, there is only one dispensing unit, which may be a pipetting needle for drawing up and discharging of a sample and a reagent. The drawing up and discharging of the sample and reagent are both performed by dispensing unit 510. When compared with the embodiment of figure 6, a dispensing unit is omitted for this embodiment, which results in a cost saving. Referring to figure 7, sample adding step S700 and reagent adding step S702 are both performed by dispensing unit 510, other structures and work flow are similar to embodiments of figure 6 and figure 7 .

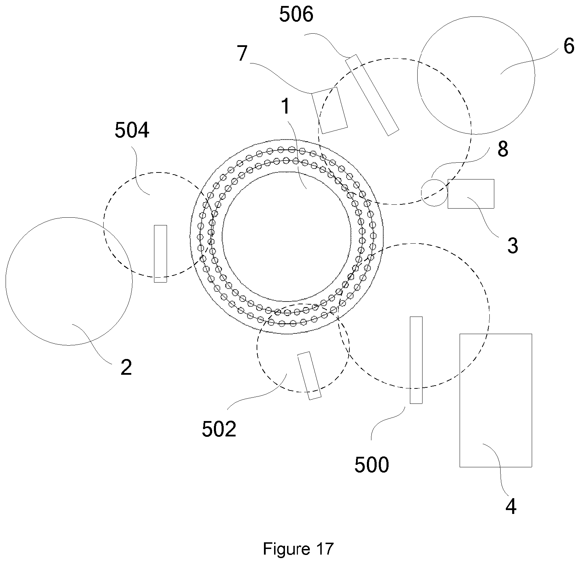

With reference to figure 17, another embodiment not being part of the invention is shown in which there is just one conveying unit. Conveying unit 520 combines the functions of the conveying cuvette of the first conveying unit 506 and the second conveying unit 500 of figure 6, which reduce the cost. The conveying unit 520 conveys the reaction vessel between the reaction vessel supply unit 4, the reaction disk 1, the B/F unit 6 and the mixing unit 7 in turn, so, the reaction vessel supply unit 4, the conveying operation position 103 and 104, the B/F unit 6 and the mixing unit 7 are located in trajectory of conveying unit 506 or intersect with the trajectory of conveying unit 506, in the work flow of figure 7 , cuvette is conveyed by conveying unit 520, other structures and work flow are similar to the embodiment of figure 6 .

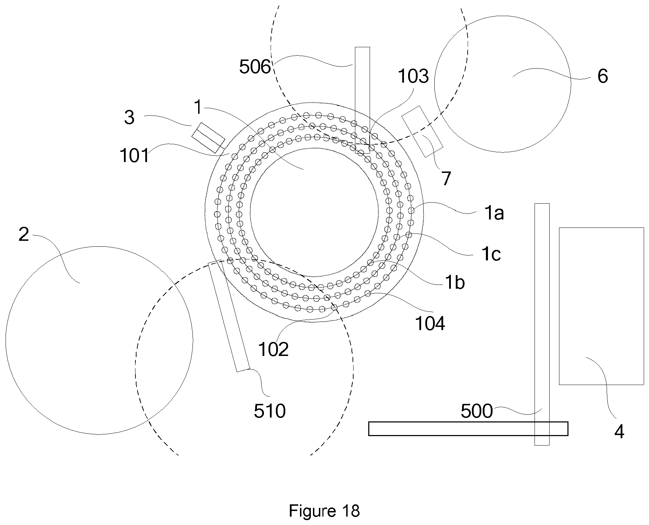

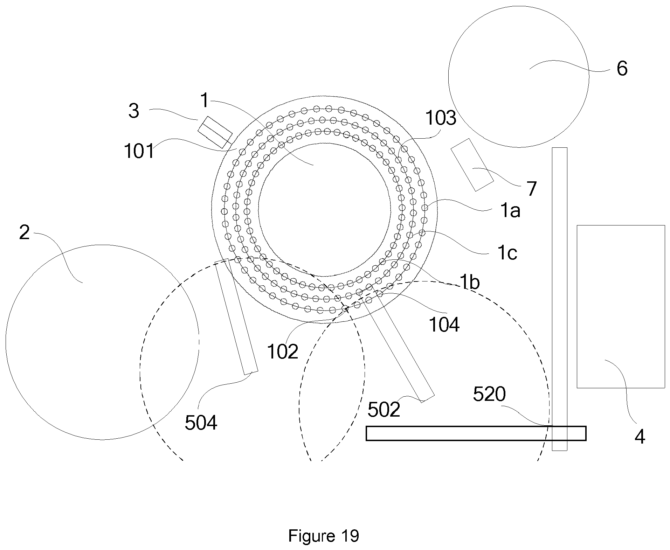

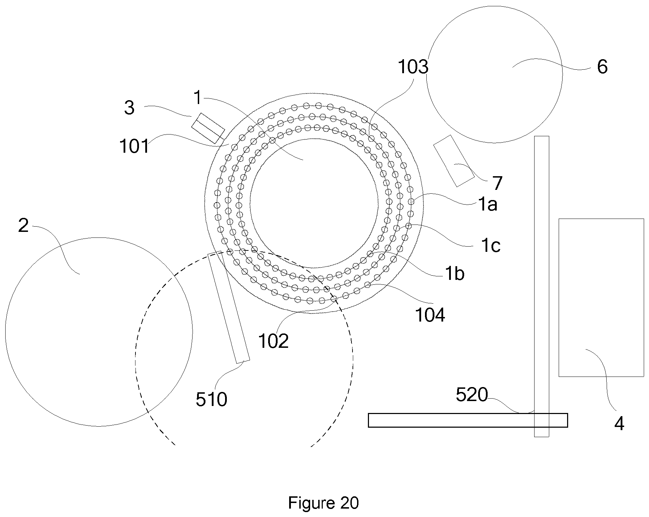

Figure 20 shows another embodiment not being part of the invention, in which there is just one dispensing unit, and just one conveying unit. Dispensing unit 510 is similar to the dispensing unit of figure 18; conveying unit 520 is similar to the conveying unit of figure 19. Other structures and the work flow are similar to embodiments of figure 6, which reduce the cost.

The reaction disk includes incubation positions. These positions are holes or slots. The reaction disk and the B/F unit are independent structures. By arranging the reaction disk and the B/F unit separately, The incubation and B/F of the reaction vessel are finished in different units, so as to avoid the incubation and B/F influencing one another Te analysis and testing is thus more flexible and at the same time as avoiding B/F affecting the incubation, it is convenient for controlling the temperature conditions and improve the work efficiency.

The present reaction disk, can further comprise the optical unit for detecting an analyte in the reaction vessel in the detection operation position and also the second incubation with added signal reagent. By integrating a plurality of functions in one structure, the system structure is simplified. Hence after the termination of the second incubation following the addition of the signal reagent to the reaction vessel, the reaction vessel is turned to the detection operation position for detecting the analyte directly. In this way, the step of conveying the reaction vessel out of the reaction disk for detecting is omitted, so testing time is saved and the test step is simplified leading to improved efficiency. In the invention, if there are multi-rings reaction disk, the detection operation position and the second incubation are on the same ring. Thus, when reaction vessel has finished the second incubation, it can be turned to the detection operation position for detection. Certainly, it is also possible that the detection operation position and the second incubation are not in the same ring, in which case, after finishing the second incubation, the reaction vessel needs only to be conveyed to the ring having the detection operation position for detection. Hence, the step of conveying the reaction vessel out of reaction disk for detecting is also omitted, which simplifies the detection step and the system structure is simplified.

Usually, the temperature for both the first incubation and the second incubation, which occurs after adding the signal reagent to the reaction vessel, are the same. Thus by arranging the first incubation and the second incubation in the same disk, the system structure is simplified, and energy is reused, because the first incubation and the second incubation are not in different disk, the energy consumption is saved. Certainly, for some luminous methods, there is no need for incubation after adding the signal reagent, and the reaction vessel can be conveyed to the detection operation position directly.

In order to achieve the two step one B/F protocol, in a specific embodiment, reference is made to figure 8. In step S800 and step S802, the sample and the first reagent are added by the dispensing unit. In step S804, the reaction vessel is conveyed to mixing unit 7 for mixing. Mixing unit may be independent from the reaction disk 1; the reaction disk can also having a mixing position for mixing. There are many kinds of mixing methods, such as, for example, ultrasonic wave, etc. In step S806, the first incubation takes place of the reaction solution consisting of the sample and the reagent. In step S808, the first incubation has terminated and the reaction vessel is conveyed to the dispensing operation position 102 where the second reagent is added. In step S810, the reaction solution after adding the second reagent is mixed. In step S812, the first incubation is performed for the reaction after adding the second reagent. In step S814, the B/F of reaction vessel is executed. In step S815, the signal reagent is added. In step S816, the second incubation is executed. In step S818, detection is performed directly after finishing the second incubation. Some analysis methods do not need a mixing operation.

As can be seen from the above description, for the present automatic analyzer, in reaction disk 1, it is possible not only to detect signals in the reaction vessel in detection operation position 101, but also to perform the second incubation of the reaction vessel after adding the signal reagent. The system structure is thus simplified. Moreover, the step of conveying the reaction vessel to the detection operation position 101 for signal detection after the second incubation with added signal reagent, and the step of conveying the reaction vessel out of reaction disk for detecting are omitted. Consequently, time for testing is saved and the efficiency is higher. In addition, the first incubation and the second incubation after adding the signal reagent to the reaction solution are arranged in the same ring thus energy is reused, because the first incubation and the second incubation are not in different disks, energy consumption is saved, and the system structure is simplified. When the reaction disk, the first incubation position may be arranged in any ring or any several rings of reaction disk 1 has has multiple rings, the second incubation position may also be arranged in any ring or any several rings of the reaction disk 1. It is preferred that the second incubation position is arranged in the ring which is close to detection unit 3. In this way, after termination of the second incubation, the reaction vessel may be conveyed by the reaction disk to the detection operation position 101 directly and the flow is easier. As mentioned above, some tests do not require incubation after adding the signal reagent. In this case, the reaction disk 1 is conveyed to the detection operation position 101 for detection directly.

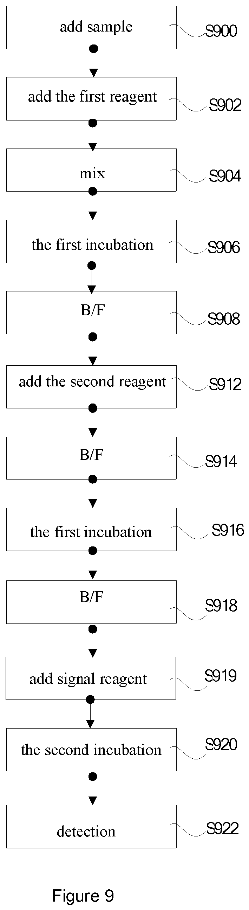

In order to achieve a two step one B/F protocol, in specific embodiment, reference is made to figure 9. In step S900 and S902, dispensing unit adds sample and reagent to the reaction vessel. In step S904, the reaction vessel is conveyed to mixing unit 7 for mixing. In step S906, the first incubation of sample and reagent is executed, in step S908, the reaction vessel is conveyed to B/F unit 6 for cleaning and separation. In step S912, the second reagent is added. In step S914, the reaction solution is mixed after adding the second reagent. In step S916, the first incubation is executed after the solution is mixed. In step S918, B/F is performed. In step S919, signal reagent is added. In step S920, the second incubation is performed. In step S922, detecting is performed. As mentioned earlier, some tests do not require incubation after adding the signal reagent. In this case the reaction disk 1 moves to the detection operation position 101 directly for detection. Some analysis methods do not need a mixing operation.

As show in figure 6, in order to supply more incubation positions, so as to improve throughput rate, there are three rings in the reaction disk 1, including an inner ring, a middle ring and an outer ring. In the reaction disk, there are two rings for the first incubation. The middle ring and the inner ring are used for the first incubation. The detection unit 3 is closed to the reaction disk, so as to facilitate detection. Because detection unit 3 is arranged out of the reaction disk 1, the detection operation is realized in the outer ring.

In addition to the embodiment described using chemiluminescence, the present invention also can apply to immunoassay analyzer of fluorescence immunoassay, electrochemiluminescence immunoassay, etc.

In embodiments of the present invention, the technical features or operational steps can be combined in any manner. Those skilled in the art will easily understand that the sequence of the method steps or actions can be changed in embodiments of the present invention. So, unless a certain order is otherwise specified, the steps given in the drawings or the detailed description are just for illustrative purposes, and not the order necessary.

In embodiments of the present invention, there may be include several steps and these steps may be embodied as executable instructions which can be performed by operation of a machine which can be operated by a general-purpose or special-purpose computer (or other electronic devices). Preferably, these steps are be operated by hardware components which include specific logic circuits for performing these steps, or operated by hardware, software and/or firmware implementation together.

As can be seen from the above analysis, these three methods all include the second incubation which is performed after adding the signal reagent, and subsequently detection. However,, some tests do not require incubation after adding the signal reagent, but can perform detection directly. In addition, the above methods may be extended to other methods, for example, by adding a third reagent, etc. The reaction vessel does not need be conveyed out of the reaction disk 1 to the detection position for detection after the second incubation is finished, so the system is simplified and the test time is saved. In accordance with the present invention, the reaction vessel can be conveyed in the reaction disk to the detection operation position 101. The reaction disk includes the first incubation position for the reaction solution. If incubation after adding the signal reagent is needed, the reaction disk may also include the second incubation position for the reaction vessel which contains added signal reagent.

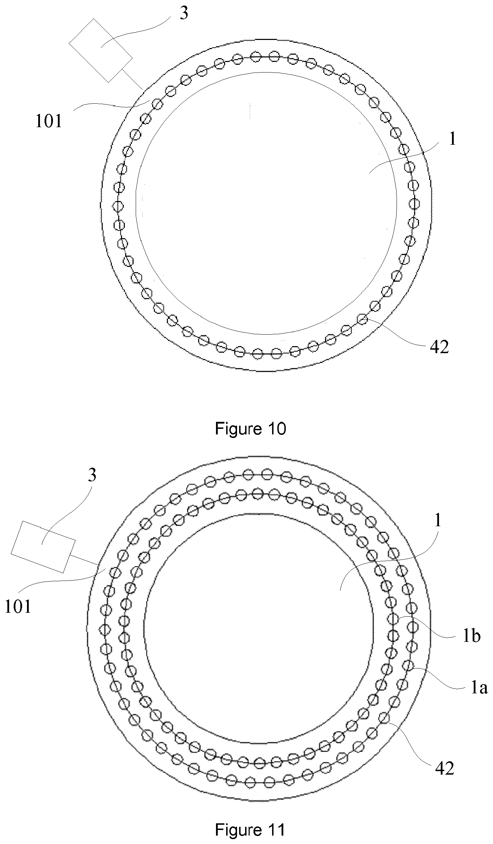

In another embodiment, please refer to figure 10, there is only one ring in the reaction disk 1, with a number of positions located thereon. These positions may be holes or slots, etc, for incubation or/and holding the reaction vessel 42, and conveying the reaction vessel 42 to a determined operation position, the operation position including a detection operation position 101. Detection unit 3 is out of the reaction disk 1. Of the reaction disk means outside of the rings of the reaction disk. When the reaction vessel 42 is turned to the detection operation position 101, the detection unit 3 detects and analysess the signal in the reaction vessel 42. The reaction disk 1 also includes a first incubation position which is for incubation of the reaction solution. The reaction disk 1 may also include a second incubation position which is for incubation of the reaction vessel 42. Certainly, the detection unit 3 may located at inner of the reaction vessel 1. Similarly, the reaction vessel 42 is detected and analysed when it is turned to the detection operation position 101. Because a plurality of functions are provided on one reaction disk, the system is simplified, the test time is saved, the test flow is simplified and the efficiency of the analysis of the automatic analyzer is improved. The incubation position and B/F position are located separately from one another so the mutual influence of B/F and incubation is avoided. It is thus easier to control the temperature of the reaction disk and the operation of B/F and improve the efficiency of incubation and B/F. In accordance with the present invention, in other embodiments, the reaction disk may include two rings. In this case, the reaction disk is turned to a specific operating position, which includes a detection operating position. The detection unit is out of the reaction disk. When incubation after adding the signal reagent is need, the outer ring may include the second incubation position, and the inner ring include the first incubation position. Clearly, if there is a blank position in the outer ring, the outer ring may also do the first incubation. The detection unit may also be located at inner of the reaction disk, then, by locating the first incubation position on the outer ring, the inner ring may include the second incubation position. If there is blank position in the inner ring the inner ring may also do the first incubation. Based on the above embodiments, the reaction disk may be arranged with multiple rings, for example, three rings or more so, more reaction vessels can be held or incubated.

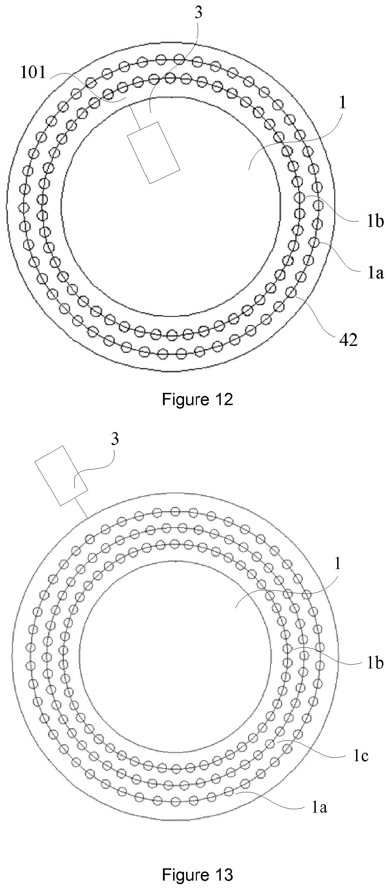

Referring to figure 11, in a specific embodiment, there are two rings in the reaction disk 1, including outer ring 1 a and inner ring 1 b. The reaction disk is rotated to specific operating position, which includes a detection operating position 101. The detection unit is 3 is outside the reaction disk. When incubation after adding the signal reagent is needed, the outer ring may include a second incubation position, and the inner ring 1 b include a first incubation position. The detection unit may also be located inside the reaction disk as shown in figure 12. The outer ring 1 a includes the first incubation position. When incubation after adding the signal reagent is needed the inner ring may also include the second incubation position.

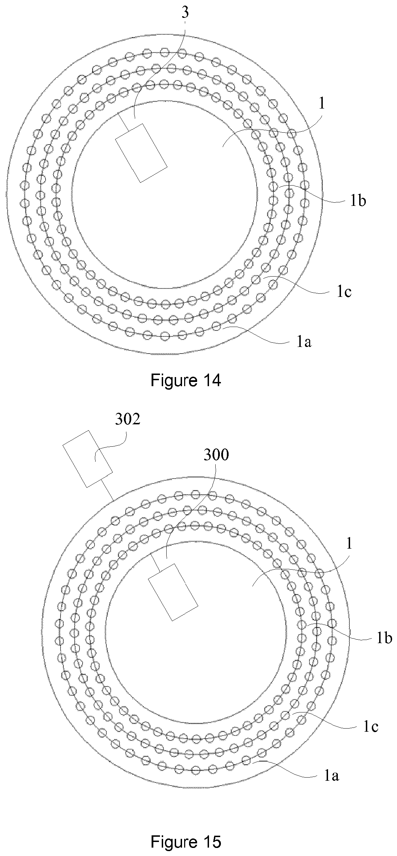

In a further embodiments shown in figure 13 there are three rings in the reaction disk 1, including outer ring 1a, inner ring 1b and middle ring 1c. The reaction disk is turned to a specific operating position, which includes a detection operating position. The detection unit is 3 is outside the reaction disk. When incubation after adding the signal reagent is needed the outer ring may include the second incubation position, while the inner ring 1 b and the middle ring 1 c include the first incubation position. In this way, the number of the incubation positions is increased. At the same time, the reaction disk may hold more reaction vessels to incubate, so the test efficiency is increased. The detection unit 3 may also be located inside the reaction disk 1 as shown in figure 14. The outer ring 1 a and the middle ring 1 c include a first incubation position. When incubation after adding the signal reagent is needed the inner ring may also include the second incubation position. Similarly, the number of incubation positions are increased, the reaction disk may hold more reaction vessels for incubation, so the test efficiency is increased. The detection unit 3 may be arranged both inside and outside the reaction disk 1. As shown in figure 15 , the reaction disk 1 is rotated to a specific operating position where there are arranged two detection units, including an outer detection unit 302 and an inner detection unit 300. The outer detection unit 302 is outer of the reaction disk 1, the inner detection unit 300 is inner of reaction disk 1. When incubation after adding the signal reagent is needed the outer ring and the inner ring may also include the second incubation position. The middle ring 1 c includes the first incubation position. Further, by increasing the number of rings of the reaction disk, more reaction vessels can be held for incubation and detection.

The present invention also provides an analyzer, including: a reaction unit for holding a reaction vessel and carrying the reaction vessel to a predetermined operation position, a detection unit, for detecting an analyte in the reaction vessel, a B/F unit, for removing the unbound components of the reaction system, a dispensing unit, for dispensing reagent or/and sample to the reaction vessel, wherein the reaction unit is provided with an incubation position for incubating solution in the reaction vessel.

By arranging the incubation and B/F separately, the influence of the B/F operation on incubation temperature is avoided. Further the B/F operation is not restricted to incubation operation. The incubation efficiency is improved and, at the same time, the test flow is more flexible. Because the incubation unit includes a first incubation position and a second incubation position, the two incubations are both incubated in the reaction disk, and the incubation efficiency is improved, so saving energy, and simplifying the system structure.

In a specific embodiment not being part of the invention, shown in figure 17, the automatic analyzer includes reaction disk 1, reagent disk 2, detection unit 3, reaction vessel supply unit 4, a second conveying unit 500, sample dispensing unit 502, reagent dispensing unit 504, a first conveying unit 506, B/F unit 6 and mixing unit 7. And the detection unit 3 of the automatic analyzer includes a detection position 8. The reaction disk has a ring structure, including at least one ring. There are a number of positions on one ring; these position may be slots or holes, etc, for incubation or/and holding the reaction vessel for a predetermined operation, while other units do the corresponding operation, for example, adding sample or reagent, or conveying. The reaction disk 1 includes an incubation position, the incubation position includes an incubation position and a second incubation position. The reagent vessel 2 is used for containing the reagent vessel, for example, the reagent bottle. The detection position 8 is independent from the reaction disk 1. It may be arranged outside the reaction disk 1, and it may also be arranged inside the reaction disk 1. The detection unit 3 is used for detecting an analyte in the reaction vessel, which is in detection position 7 using the detection unit, such as, for example, a photometer, to detect the optical signal to test the content of the analyte. The reaction vessel supply unit 4 is used for holding reaction vessels, for example, a cuvette. The second conveying unit 500 is used for conveying the reaction vessel between the reaction disk 1 and the reaction vessel supply unit 4. The dispensing unit includes sample dispensing unit 502, which is used for dispensing sample, and the reagent dispensing unit 504. The sample dispensing unit 502 may be a sample needle which is used for drawing and discharging the sample; the reagent dispensing unit 504 may be a reagent needle which is used for drawing and discharging the reagent. The reaction disk 1, mixing unit 7, B/F unit 6 and detection unit 3 are in the trajectory of the first conveying unit 506, which is used for conveying the reaction vessel between the reaction disk 1 and the B/F unit 6. It may also be used for conveying the reaction vessel between the reaction disk 1 and the mixing unit 7, and it may also be used for conveying the reaction vessel between the reaction disk 1 and the detection position 8. The B/F unit 6 is used to remove the unbound components of the reaction system. The mixing unit 7 is used to mix the reaction solution, however, some analysis methods do not need mixing. During the analysis, the reaction vessel is conveyed from the reaction disk 1 to the detection unit 8, then, the detection unit detects the analyte in the reaction vessel, which is in the detection position 8. Because the optical signal from the solution in the reaction vessel is very low, it can easily be disturbed by the environment. The optical signal detection needs a good dark environment. The dark environment is easier to form in structure by arranging the detection position 8 separately. On the other hand, the need for the detection to provide incubation and conveying is avoided. Because the second incubation after adding signal reagent is finished in the reaction disk 1, the detection unit 3 does not need to provide the second incubation function. In another embodiment, the detection position 8 is not arranged separately, however, detecting the sample in the reaction disk 1, that is, in the reaction vessel on the reaction disk is detected in the detection position 101 by the detection unit, which is similar to figure 6.

In a specific test, the adding of the sample and reagent at the reaction disk 1, with the sample being added by sample dispensing unit 502 and the reagent being added by reagent dispensing unit 504, as described above, can be performed such that the first the reagent is added and then, the sample. The reaction vessel is then conveyed to the mixing unit 7 for mixing by the first conveying unit 506, then the reaction vessel is conveyed to the first incubation position of the reaction disk 1 for the first incubation by the first conveying unit 506. When the first incubation is finished, the reaction vessel is conveyed to the B/F unit 6 for cleaning and separation by the first conveying unit 506 and the signal reagent then added. As mentioned earlier, the signal reagent can be added at another position. The reaction vessel is then conveyed to the second incubation position of the reaction disk 1 for the second incubation by the first conveying unit 506. When the second incubation is finished, the reaction vessel is conveyed to detection unit 3 for detecting by the first conveying unit 506.

The reaction disk has a ring structure, including at least one ring, which has a first incubation position and a second incubation position. Certainly, two detection units 3 may also be provided, then, the reaction vessel accordingly can be subjected to optical detection, increasing the numbers of detection and improving the analysis efficiency of analyzer. The mixing unit 7 may be arranged separately, and also may be arranged in the reaction disk 1. The detection unit 3 may be arranged separately as in the previous embodiments, and also may be arranged around the reaction disk. The reaction vessel is detected in detection operation position by detection unit 3.

As mentioned earlier, in the specific implementation process, considering factors of optimization of combination test efficiency, the whole size of machine and cost, etc, the number of the dispensing unit and the conveying unit can be increased or reduced, the dispensing unit and the conveying unit can be flexible assembled by different number, for example, just one dispensing unit and just one conveying unit, or three dispensing units and two conveying units, etc.

The present invention also provides a sample analysis method, including: a dispensing step of adding the sample and reagent to the reaction vessel, an incubation step of incubating the reaction vessel in the reaction unit, an adding signal reagent step of adding signal reagent to the reaction vessel, a detection step of conveying the reaction vessel to the detection operation position by the reaction unit, then, detecting the analyte in the reaction vessel in the detection operation position by the detection unit.

The present incubation step is executed in the reaction disk, the B/F is executed in the B/F unit. Since the incubation step and the B/F step are executed in different structures, any influence between the B/F step and the incubation step is avoided, which is convenient to control the temperature condition of incubation and improve the work efficiency. In present invention, not only is the incubation step executed in the reaction disk, but the detection step is also executed in the detection operation position by the detection unit. By combining many kinds of functions together, the system structure is simplified and the reaction vessel does not need to be conveyed to a detection position which is outside the reaction disk for detection and analysis. The operation which conveys the reaction vessel out of the reaction disk for detection after the second incubation has finished is omitted, thereby saving test time, simplifying the test step and improving the efficiency of the automatic analyzer. In present invention, the first incubation step and the second incubation step are executed are in the same disk, so that the system structure is simplified and energy is reused, because the incubations do not need in two different disks, the energy cost is saved. Certainly, for some luminous methods, it is not necessary to incubate after adding the signal reagent, but detection in the reaction vessel can be performed directly.

The present invention also provides a sample analysis method, including, adding sample and reagent to a reaction vessel, a first incubation step by subjecting the reaction vessel in the reaction unit to a first incubation, a B/F step by removing unbound components of reaction system, a signal reagent adding step by adding a signal reagent to the reaction vessel, a second incubation step by subjecting the reaction vessel in the reaction unit to a second incubation, a detection step by detecting an analyte in the reaction vessel.

The incubation step of the present invention is executed in the reaction disk, while the B/F step is executed in the B/F unit, so the incubation step and the B/F step are executed in different structures, avoiding the influence between the B/F step and the incubation step, which is convenient to control the temperature condition of incubation, thereby improving the work efficiency. In accordance with the present invention, the first incubation step and the second incubation step are both executed in the same disk. The system structure is thus simplified and the energy is reused, because there is no need to incubate in a different disk resulting in an energy cost saving. In accordance with the present invention, the detection unit is arranged separately from the reaction disk as this is more convenient for providing a dark environment in structure. At the same time, the detection is not restricted to the incubation of the reaction disk as the detection step is finished in the detection unit which is out of the reaction disk. Certainly, the detection unit may also be located around the reaction disk, so that after the second incubation is finished the reaction vessel is conveyed to the detection operation position and the detection of the reaction vessel is finished in the detection operation position by the detection unit.

The present invention is described with reference to specific embodiments, but the present invention is not limited to these embodiments. Those having skill in the art will understand that various modifications, equivalent replacements, changes, etc, can be made , and providing these transformations do not departing from the invention , these are all within the scope of protection. In addition, "one embodiment" or "another embodiment" means different embodiment, however, the whole or part of these embodiments can be combined to a embodiment.

The above description describes several embodiments for the present invention and is very specific and detailed, but should not be understood to restrict to this invention. It should be noted that those having skill in the art, will understand that certain changes and modifications can be made based on the present invention and without departing from the inventive concept, and these are all within the scope of protection. The scope of the present invention should, therefore, be determined only by the following claims.

## Claims

1. An automatic analyzer, including:
a single reaction disk (1) for holding a reaction vessel (42) and carrying the reaction vessel to a predetermined operation position, the operation position including a dispensing operation position (102) and a detection operation position (101) on the single reaction disk (1);
a detection unit (3), for detecting an analyte in the reaction vessel (42) in the single reaction disk (1) at the detection operation position;
a single B/F unit (6), for removing unbound components of reaction system;
a dispensing unit (502, 504, 510), for dispensing a reagent and/or a sample to the reaction vessel (42);
a reaction vessel supply unit (4), for holding reaction vessels (42); and
a conveying unit (506,500,520), for conveying the reaction vessel between the single reaction disk (1), the single B/F unit (6) and the reaction vessel supply unit (4), wherein the conveying unit (520) includes a first convey unit (506) and a second convey unit (500), the first conveying unit (506) for conveying the reaction vessel (42) between the single reaction disk (1) and the single B/F unit (6), the second conveying unit (500) for conveying the reaction vessel (42) between the single reaction disk (1) and the reaction vessel supply unit (4);
wherein the single reaction disk (1) and the single B/F unit (6) are independent structures, and the single B/F unit (6) is disposed outside and separated from the single reaction disk (1) with sufficient spacing to avoid cross-influence between the single B/F unit (6) and the single reaction disk (1), and the single reaction disk (1) is provided with a first incubation position and a second incubation position which are distinct from each other, wherein the single reaction disk (1) is configured to incubate a solution with a reagent and a sample in the reaction vessel at the first incubation position to form a reaction solution, the first conveying unit (506) is configured to convey the reaction solution in the reaction vessel to the single B/F unit (6) to form a cleaned reaction solution, and convey the cleaned reaction solution with a signal reagent in the reaction vessel to the single reaction disk (1), and the single reaction disk (1) is further configured to incubate the cleaned reaction solution with the signal reagent in the reaction vessel at the second incubation position to form an analyte, and convey the analyte to the detection operation position (101) on the single reaction disk (1) for detection.

2. The analyzer of claim 1, wherein the single B/F unit (6) is disposed in a disjoint arrangement with the single reaction disk (1).

3. The analyzer of claim 1, wherein the single reaction disk (1) includes at least two rings (1a, 1b), wherein the detection unit (3) is arranged outside and/or inside the single reaction disk (1), and wherein an outer ring (1a) and/or an inner ring (1b) of the single reaction disk (1) includes the second incubation position.

4. The analyzer of claim 1, wherein the single reaction disk (1) includes at least three rings (la, 1b, 1c) with an outer ring (1a) being arranged at the outside, a middle ring (1c) being arranged in the middle and an inner ring (1b) being arranged inside, the outer ring (1a) including the second incubation position, the inner ring (1b) and the middle ring (1c) including the first incubation position, wherein the detection unit (3) is arranged outside the single reaction disk (1).

5. The analyzer of any of claims 1-4, wherein the automatic analyzer includes a mixing unit (7) for mixing the solution in the reaction vessel (42).

6. The analyzer of claim 1, wherein the dispensing unit (510) includes a sample dispensing unit (502) and a reagent dispensing unit (504), the sample dispensing unit (502) for dispensing sample to the reaction vessel (42), the reagent dispensing unit (504) for dispensing reagent to the reaction vessel (42).

7. The analyzer of claim 1, wherein the dispensing unit is a single dispensing unit for dispensing sample and reagent to the reaction vessel (42).

8. A sample analysis method, including
a dispensing step (S700, S702, S800, S802, S808, S900, S902, S912), for dispensing a sample and a reagent to a reaction vessel in a single reaction disk;
a first incubation step (S706, S806, S812: S906, S916), for a first incubation of the reaction vessel in the single reaction disk;
a B/F step (S708, S814, S908, S914, S918), for removing unbound components of a reaction system in a single B/F unit, and wherein the single reaction disk and the single B/F unit are independent structures, and the single B/F unit is disposed outside the single reaction disk and separated from the single reaction disk with sufficient spacing to avoid cross-influence between the single B/F unit (6) and the single reaction disk;
an adding signal reagent step (S709, S815, S919), for adding signal reagent to the reaction vessel;
a second incubation step (S710, S816, S920), for a second incubation of the reaction vessel in the single reaction disk; and
a detection step (S712, S818, S922), for detecting an analyte in the reaction vessel at a detection operation position on the single reaction disk;
wherein the single reaction disk is provided with a first incubation position for the first incubation step and a second incubation position for the second incubation step which are distinct from each other, a solution with a reagent and a sample in the reaction vessel is incubated at the first incubation position to form a reaction solution, the reaction solution in the reaction vessel is conveyed to the single B/F unit to form a cleaned reaction solution, the cleaned reaction solution with the signal reagent in the reaction vessel is conveyed to the single reaction disk and incubated at the second incubation position to form the analyte, and the analyte is conveyed to the detection operation position on the single reaction disk for detection.

9. The method of claim 8, wherein the method further includes: conveying, by a conveying unit, the reaction vessel between the single reaction disk and the single B/F unit.

## Patentansprüche

1. Ein automatisches Analysegerät mit:
einer einzigen Reaktionsplatte (1) zum Halten eines Reaktionsbehälters (42) und zum Transport des Reaktionsbehälters in eine vorbestimmte Arbeitsposition, wobei die Arbeitsposition eine Abgabearbeitsposition (102) und eine Erfassungsarbeitsposition (101) auf der einzigen Reaktionsplatte (1) umfasst,
eine Erfassungseinheit (3) zur Erfassung eines Analyts im Reaktionsbehälter (42) auf der einzigen Reaktionsplatte (1) in Erfassungsarbeitsposition,
einer einzigen B/F-Einheit (6) zur Entfernung ungebundener Komponenten aus dem Reaktionssystem,
einer Abgabeeinheit (502, 504, 510) zur Abgabe eines Reagens und/oder einer Probe an den Reaktionsbehälter (42),
eine Reaktionsbehälter-Versorgungseinheit (4) zum Halten der Reaktionsbehälter (42) und
einer Fördereinheit (506, 500, 520) zum Transport des Reaktionsbehälters zwischen der einzigen Reaktionsplatte (1), der einzigen B/F-Einheit (6) und der Reaktionsbehälter-Versorgungseinheit (4), wobei die Fördereinheit (520) eine erste Fördereinheit (506) und eine zweite Fördereinheit (500) umfasst, die erste Fördereinheit (506) zum Transport des Reaktionsbehälters (42) zwischen einziger Reaktionsplatte (1) und einziger B/F-Einheit (6), die zweite Fördereinheit (500) zum Transport des Reaktionsbehälters (42) zwischen einziger Reaktionsplatte (1) und Reaktionsbehälter-Versorgungseinheit (4),
wobei einzige Reaktionsplatte (1) und einzige B/F-Einheit (6) unabhängige Strukturen sind und die einzige B/F-Einheit (6) außerhalb und getrennt von der einzigen Reaktionsplatte (1) mit ausreichend Platz angeordnet ist, um Quereinflüsse zwischen einziger B/F-Einheit (6) und einziger Reaktionsplatte (1) zu vermeiden, und die einzige Reaktionsplatte (1) eine erste Inkubationsposition und eine zweite Inkubationsposition aufweist, die sich unterscheiden, wobei die einzige Reaktionsplatte (1) zur Inkubation einer Lösung mit einem Reagens und einer Probe in einem Reaktionsbehälter in der ersten Inkubationsposition zur Bildung einer Reaktionslösung ausgelegt ist, die erste Fördereinheit (506) zum Transport der Reaktionslösung im Reaktionsbehälter zur einzigen B/F-Einheit (6) ausgelegt ist, um eine bereinigte Reaktionslösung zu bilden und die bereinigte Reaktionslösung mit einem Signalreagens im Reaktionsbehälter an die einzige Reaktionsplatte (1) zu transportieren, und die einzige Reaktionsplatte (1) weiterhin zur Inkubation der bereinigten Reaktionslösung mit dem Signalreagens im Reaktionsbehälter in der zweiten Inkubationsposition zur Bildung eines Analyts ausgelegt ist, sowie zum Transport des Analyts in die Erfassungsarbeitsposition (101) der einzigen Reaktionsplatte (1) zur Erfassung.

2. Das Analysegerät gemäß Anspruch 1, wobei die B/F-Einheit (6) in einer von der einzigen Reaktionsplatte (1) getrennten Vorrichtung untergebracht ist.

3. Das Analysegerät gemäß Anspruch 1, wobei die einzige Reaktionsplatte (1) mindestens zwei Ringe (1a, 1b) umfasst, wobei die Erfassungseinheit (3) außerhalb und/oder innerhalb der einzige Reaktionsplatte (1) angeordnet ist und wobei ein äußerer Ring (1a) und/oder ein innerer Ring (1b) der einzigen Reaktionsplatte (1) die zweite Inkubationsposition umfasst.

4. Das Analysegerät gemäß Anspruch 1, wobei die einzige Reaktionsplatte (1) mindestens drei Ringe (1a, 1b, 1c) umfasst, wobei ein äußerer Ring (1a) außen, ein mittlerer Ring (1c) in der Mitte und ein innerer Ring (1b) innen angeordnet ist, der äußere Ring (1a) die zweite Inkubationsposition enthält, der innere Ring (1b) und der mittlere Ring (1c) die erste Inkubationsposition enthalten und wobei die Erfassungseinheit (3) außerhalb der einzigen Reaktionsplatte (1) angeordnet ist.

5. Das Analysegerät gemäß einem der Ansprüche 1 bis 4, wobei das automatische Analysegerät eine Mischeinheit (7) zum Mischen der Lösung im Reaktionsbehälter (42) umfasst.

6. Das Analysegerät gemäß Anspruch 1, wobei die Abgabeeinheit (510) eine Probeabgabeeinheit (502) und eine Reagensabgabeeinheit (504) enthält, die Probeabgabeeinheit (502) zur Abgabe der Probe in den Reaktionsbehälter (42) und die Reagensabgabeeinheit (504) zur Abgabe des Reagens in den Reaktionsbehälter (42).

7. Das Analysegerät gemäß Anspruch 1, wobei die Abgabeeinheit eine einzige Abgabeeinheit zur Abgabe von Probe und Reagens in den Reaktionsbehälter (42) ist.

8. Ein Verfahren zur Probeanalyse mit:
einem Abgabeschritt (S700, S702, S800, S802, S808, S900, S902, S912) zur Abgabe einer Probe und eines Reagens in einen Reaktionsbehälter in einer einzigen Reaktionsplatte,
einem ersten Inkubationsschritt (S706, S806, S812; S906, S916) für eine erste Inkubation des Reaktionsbehälters in der einzigen Reaktionsplatte,
einem B/F-Schritt (S708, S814, S908, S914, S918) zur Entfernung ungebundener Komponenten aus einem Reaktionssystem in einer einzigen B/F-Einheit, wobei die einzige Reaktionsplatte und die einzige B/F-Einheit unabhängige Strukturen sind und die einzige B/F-Einheit außerhalb der einzigen Reaktionsplatte untergebracht und getrennt von der einzigen Reaktionsplatte ist, mit ausreichend Abstand, um Quereinflüsse zwischen einziger B/F-Einheit (6) und einziger Reaktionsplatte zu vermeiden,
einem Schritt der Hinzugabe von Signalreagens (S709, S815, S919) zur Hinzugabe von Signalreagens in den Reaktionsbehälter,
einem zweiten Inkubationsschritt (S710, S816, S920) für eine zweite Inkubation des Reaktionsbehälters in der einzigen Reaktionsplatte und
einem Erfassungsschritt (S712, S818, S922) zur Erfassung eines Analyts im Reaktionsbehälter in einer Erfassungsarbeitsposition der einzigen Reaktionsplatte,
wobei die einzige Reaktionsplatte eine erste Inkubationsposition für den ersten Inkubationsschritt aufweist und eine zweite Inkubationsposition für den zweiten Inkubationsschritt, die sich unterscheiden, eine Lösung mit einem Reagens und einer Probe im Reaktionsbehälter in der ersten Inkubationsposition inkubiert wird, um eine Reaktionslösung zu erhalten, die Reaktionslösung im Reaktionsbehälter an die einzige B/F-Einheit gesandt wird, um eine bereinigte Reaktionslösung zu bilden, die bereinigte Reaktionslösung mit dem Signalreagens im Reaktionsbehälter an die einzige Reaktionsplatte befördert und in der zweiten Inkubationsposition inkubiert wird, um das Analyt zu bilden, und das Analyt in die Erfassungsarbeitsposition der einzigen Reaktionsplatte zur Erfassung gebracht wird.

9. Das Verfahren gemäß Anspruch 8, wobei das Verfahren weiterhin Folgendes umfasst: Transport des Reaktionsbehälters über eine Fördereinheit zwischen einziger Reaktionsplatte und einziger B/F-Einheit.

## Revendications

1. Analyseur automatique, incluant :
un unique disque de réaction (1) destiné à contenir un récipient de réaction (42) et à transporter le récipient de réaction jusqu'à une position de fonctionnement prédéterminée, la position de fonctionnement incluant une position de fonctionnement de distribution (102) et une position de fonctionnement de détection (101) sur l'unique disque de réaction (1) ;
une unité de détection (3), destinée à détecter un analyte dans le récipient de réaction (42) dans l'unique disque de réaction (1) à la position de fonctionnement de détection ;
une unique unité B/F (Bound/Free, fraction liée/fraction libre) (6), destinée à éliminer les composants non liés du système réactionnel ;
une unité de distribution (502, 504, 510), destinée à distribuer un réactif et/ou un échantillon dans le récipient de réaction (42) ;
une unité de fourniture de récipients de réaction (4), destinée à contenir des récipients de réaction (42) ; et
une unité d'acheminement (506, 500, 520), destinée à acheminer le récipient de réaction entre l'unique disque de réaction (1), l'unique unité B/F (6) et l'unité de fourniture de récipients de réaction (4), où l'unité d'acheminement (520) inclut une première unité d'acheminement (506) et une seconde unité d'acheminement (500), la première unité d'acheminement (506) étant destinée à acheminer le récipient de réaction (42) entre l'unique disque de réaction (1) et l'unique unité B/F (6), la seconde unité d'acheminement (500) étant destinée à acheminer le récipient de réaction (42) entre l'unique disque de réaction (1) et l'unité de fourniture de récipients de réaction (4) ;
où l'unique disque de réaction (1) et l'unique unité B/F (6) sont des structures indépendantes, et l'unique unité B/F (6) est disposée à l'extérieur et à l'écart de l'unique disque de réaction (1) avec un espace suffisant pour éviter toute interférence mutuelle entre l'unique unité B/F (6) et l'unique disque de réaction (1), et l'unique disque de réaction (1) est pourvu d'une première position d'incubation et d'une seconde position d'incubation qui sont distinctes l'une de l'autre, où l'unique disque de réaction (1) est configuré pour incuber une solution avec un réactif et un échantillon dans le récipient de réaction à la première position d'incubation afin de former une solution réactionnelle, la première unité d'acheminement (506) est configurée pour acheminer la solution réactionnelle dans le récipient de réaction vers l'unique unité B/F (6) afin de former une solution réactionnelle épurée, et pour acheminer la solution réactionnelle épurée avec un réactif signal dans le récipient de réaction vers l'unique disque de réaction (1), et l'unique disque de réaction (1) est en outre configuré pour incuber la solution réactionnelle épurée avec le réactif signal dans le récipient de réaction à la seconde position d'incubation afin de former un analyte, et pour acheminer l'analyte vers la position de fonctionnement de détection (101) sur l'unique disque de réaction (1) pour une détection.

2. Analyseur selon la revendication 1, dans lequel l'unique unité B/F (6) est disposée selon un agencement séparé par rapport à l'unique disque de réaction (1).

3. Analyseur selon la revendication 1, dans lequel l'unique disque de réaction (1) inclut au moins deux anneaux (1a, 1b), où l'unité de détection (3) est agencée à l'extérieur et/ou à l'intérieur de l'unique disque de réaction (1), et où un anneau extérieur (1a) et/ou un anneau intérieur (1b) de l'unique disque de réaction (1) incluent la seconde position d'incubation.

4. Analyseur selon la revendication 1, dans lequel l'unique disque de réaction (1) inclut au moins trois anneaux (1a, 1b, 1c), un anneau extérieur (1a) étant agencé à l'extérieur, un anneau central (1c) étant agencé au milieu et un anneau intérieur (1b) étant agencé à l'intérieur, l'anneau extérieur (1a) incluant la seconde position d'incubation, l'anneau intérieur (1b) et l'anneau central (1c) incluant la première position d'incubation, où l'unité de détection (3) est agencée à l'extérieur de l'unique disque de réaction (1).

5. Analyseur selon l'une quelconque des revendications 1 à 4, où l'analyseur automatique inclut une unité de mélange (7) destinée à mélanger la solution dans le récipient de réaction (42).

6. Analyseur selon la revendication 1, dans lequel l'unité de distribution (510) inclut une unité de distribution d'échantillon (502) et une unité de distribution de réactif (504), l'unité de distribution d'échantillon (502) étant destinée à distribuer un échantillon dans le récipient de réaction (42), l'unité de distribution de réactif (504) étant destinée à distribuer un réactif dans le récipient de réaction (42).

7. Analyseur selon la revendication 1, dans lequel l'unité de distribution est une unique unité de distribution destinée à distribuer un échantillon et un réactif dans le récipient de réaction (42).

8. Procédé d'analyse d'échantillon, incluant
une étape de distribution (S700, S702, S800, S802, S808, S900, S902, S912), afin de distribuer un échantillon et un réactif dans un récipient de réaction dans un unique disque de réaction ;
une première étape d'incubation (S706, S806, S812, S906, S916), pour une première incubation du récipient de réaction dans l'unique disque de réaction ;
une étape B/F (S708, S814, S908, S914, S918), afin d'éliminer les composants non liés d'un système réactionnel dans une unique unité B/F, et où l'unique disque de réaction et l'unique unité B/F sont des structures indépendantes, et l'unique unité B/F est disposée à l'extérieur de l'unique disque de réaction et à l'écart de l'unique disque de réaction avec un espace suffisant pour éviter toute interférence mutuelle entre l'unique unité B/F (6) et l'unique disque de réaction ;
une étape d'ajout de réactif signal (S709, S815, S919), afin d'ajouter un réactif signal dans le récipient de réaction ;
une seconde étape d'incubation (S710, S816, S920), pour une seconde incubation du récipient de réaction dans l'unique disque de réaction ; et
une étape de détection (S712, S818, S922), afin de détecter un analyte dans le récipient de réaction à une position de fonctionnement de détection sur l'unique disque de réaction ;
où l'unique disque de réaction est pourvu d'une première position d'incubation pour la première étape d'incubation et d'une seconde position d'incubation pour la seconde étape d'incubation qui sont distinctes l'une de l'autre, une solution avec un réactif et un échantillon dans le récipient de réaction est incubée à la première position d'incubation afin de former une solution réactionnelle, la solution réactionnelle dans le récipient de réaction est acheminée vers l'unique unité B/F afin de former une solution réactionnelle épurée, la solution réactionnelle épurée avec le réactif signal dans le récipient de réaction est acheminée vers l'unique disque de réaction et incubée à la seconde position d'incubation afin de former l'analyte, et l'analyte est acheminé vers la position de fonctionnement de détection sur l'unique disque de réaction pour une détection.

9. Procédé selon la revendication 8, où le procédé inclut en outre : le fait d'acheminer, au moyen d'une unité d'acheminement, le récipient de réaction entre l'unique disque de réaction et l'unique unité B/F.
